# EUROPÄISCHE PATENTSCHRIFT

(11) **EP 0 752 585 B1**
(45) Veröffentlichungstag und Bekanntmachung des Hinweises auf die Patenterteilung: **02.05.2003**
(21) Anmeldenummer: 96109119.6
(22) Anmeldetag: 07.06.1996
(51) Int. Cl.: G01N 33/536, G01N 33/53, G01N 21/47, G01N 33/68

(54) **Antigenüberschussfreie nephelometrische und turbidimetrische Proteinbestimmungen**
Nephelometric and turbidimetric protein assays free from excess antigen
Essais de protéine néphélométriques et turbidimétriques sans excès d'antigène

(30) Priorität: 05.07.1995 DE 19524414
(43) Veröffentlichungstag der Anmeldung: 08.01.1997
(73) Patentinhaber: Dade Behring Marburg GmbH, 35001 Marburg (DE)
(72) Erfinder: Tuengler, Peter, Dr., 35041 Marburg (DE)

(56) Entgegenhaltungen:
- US-A- 4 157 871
- US-A- 4 204 837
- CLINICAL CHEMISTRY, Bd. 20, Nr. 8, 1974, WASHINGTON DC, Seiten 1055-1061, XP002064410 TIFFANY ET AL.: "Specific protein analysis by light-scatter measurement with a miniature centrifugal fast analyzer"

## Beschreibung

Die Erfindung betrifft das Gebiet der Proteinbestimmungen in homogener Lösung mittels Antigen-vermittelter Präzipitation durch Antikörper oder mit Antikörpern beschichteten Latexmaterialien und anschließender optischer Messung der Präzipitationsreaktion durch eine nephelometrische oder turbidimetrische Messung.

Wie durch die bekannte Reaktionskurve von Heidelberger und Kendall für die Immunpräzipitation ausgedrückt, können solche Reaktionen doppeldeutig sein, d.h. nach Überschreiten eines Maximalwertes tritt bei hohen Antigenkonzentrationen eine Signalabnahme auf, deren Wert in einer Messung nicht von dem Signal einer niedrigeren Antigenkonzentration differenziert werden kann.

Da dieses Problem der Messung bei Antigenüberschuß eine entscheidende Limitierung für die bekannten nephelometrischen und turbidimetrischen Meßverfahren darstellt, hat es bereits eine Reihe von Lösungsvorschlägen gegeben. So gibt es kinetische Verfahren, die durch die Auswertung der Reaktionskinetik (1. Ableitung) das Vorliegen eines Antigenüberschusses feststellen und dann eine entsprechende Nachmessung veranlassen (ANDERSON, US 4,157,871) oder die durch entsprechende technische Schritte eine eindeutige Auswertung der Kurve erlauben (DE-A- 33 47 162).

Ferner wurde versucht, das Problem dadurch zu lösen, daß bei der sogenannten "Nachstartmethode" eine definierte Menge einer Standardpräparation eines Analyten nachdosiert wird und der weitere Reaktionsverlauf aufgenommen wird.

Ein prinzipieller Nachteil der kinetischen Verfahren liegt in der Tatsache, daß eine Meßwertaufnahme über die gesamte Inkubationszeit notwendig ist. Dadurch wird die Meßoptik entsprechend in dieser Zeit blockiert und nur ein geringer Probendurchsatz zugelassen. Entsprechend speicherintensiv ist auch die Auswertung. Die Nachstartmethode hat diesen Nachteil nicht, ist aber durch die Notwendigkeit eines entsprechenden, wohldefinierten Standards mit höheren Testkosten verbunden.

Der vorliegenden Erfindung lag somit die Aufgabe zugrunde, eine Meßmethode, die ohne Testkostenerhöhung und mit hohem Probendurchsatz Antigen-überschußfreie Testschemata zuläßt.

Die Lösung dieser Aufgabe erfolgt im wesentlichen durch die Bereitstellung der in den Patentansprüchen beschriebenen Ausführungsformen.

Grundlage der Erfindung ist die Beobachtung, daß bei Proben mit derart hoher Antigenkonzentration, die ein entsprechend zu niedriges Signal erzeugen, durch Verdünnung der Probe vor dem Zugeben zum Test immer ein korrektes Ergebnis erzielt wird. Konsequenterweise werden in der Regel an automatisierten Geräte kritische Proben, bzw. Proben bei kritischen Testen mehrfach in unterschiedlichen Probenverdünnungen untersucht und die Ergebnisse anschließend manuell auf Validität überprüft. Das reduziert jedoch den Probendurchsatz entsprechend und erhöht die Testkosten pro untersuchte Probe.

Es wurde nun gefunden, daß die der Anmeldung zugrunde liegende Aufgabe dadurch gelöst werden kann, daß zunächst ein Aliquot der Probenmenge (bzw. der vor dem Test erstellten Probenverdünnung) in dem Test mit vollständigem Reagenzgehalt einer Inkubation unterzogen wird und nach einer bestimmten Reaktionszeit ein Meßwert (bzw. eine Meßwertdifferenz zum Startwert in einer Fixed-time-Messung) aufgenommen wird. Erst dann wird der Rest der Probe (bzw. Probenverdünnung) dem gleichen Reaktionsansatz hinzugefügt und dann dieser einem weiteren Inkubationsschritt und einer analogen Messung unterworfen.

Das Aliquot kann im Verhältnis 1 - 25 %, bevorzugterweise 2 - 20 %, ganz bevorzugterweise 5 - 10 % zur Gesamtprobenmenge stehen.

Bei einer Probe mit einer solch hohen Antigenkonzentration, welche in einem konventionellen Test einen Antigenüberschußeffekt hervorruft, wird bei dem genannten modifizierten Ansatz in der Vorreaktion aufgrund der deutlich niedrigeren Probenmenge ein deutliches Meßsignal entstehen, welches aufgrund einer Schwellenwertbetrachtung vom Analysengerät schnell, einfach und sicher detektiert werden kann. Proben, die auch im konventionellen Test problemlos und korrekt auf dem aufsteigenden Ast der Heidelberger-Kendall-Kurve ausgewertet werden können, liefern in der Vorreaktion nur ein schwaches bzw. kein Meßsignal, d.h. durch Unterschreitung des Schwellenwertes erkennt das Analysengerät hier den Normalfall.

In einer weiteren vorteilhaften Ausführungsform kann das Analysengerät durch Anlegen zweier Eichkurven für einen Test (für die Vor- und die Hauptreaktion) eine quantitative Auswertung ohne Nachmessung auch von extremen Proben vornehmen. Im Regelfall wird bei solchen, in der Regel nicht zu häufig auftretenden Proben keine Quantifizierung des vorhandenen Meßsignal vorgenommen, sondern Nachmessungen, bis die Hauptreaktion quantifizierbar ist. Die reine Schwellenwertbetrachtung erlaubt wiederum bei der Vorreaktion in der Regel deutlich kürzere Inkubationszeiten als bei der für die Quantifizierung des Meßsignal notwendigen Hauptreaktion, so daß der mögliche Testdurchsatz am Analysengerät nur geringfügig reduziert (und nicht z.B. halbiert) wird.

Die folgenden Beispiele dienen der Erläuterung der Erfindung.

### Beispiel 1:

### Bestimmung von Albumin im Urin

5 µL einer Urinprobe, welche 1:5 mit N-Diluenz (Best. Nr. OUMT, Behringwerke AG) vorverdünnt wurde, wird mit 55 µL N-Reaktionspuffer (Best. Nr. OUMS, Behringwerke AG) in eine Reaktionsküvette gegeben. Die Reaktion wird durch Zugabe von 40 µL Reagenz (N-Antiserum gegen Human-Albumin, Best. Nr. OSAL, Behringwerke AG) gestartet, welches zusammen mit 100 µL Reaktionspuffer (Best. Nr. OUMS, Behringwerke AG), ebenfalls in die Küvette gegeben wird. Der Ansatz wird durchmischt und ein Startsignal aufgenommen; nach exakt 2 Minuten wird eine weitere Messung durchgeführt, die Differenz zum Startsignal stellt das Signal der Vorreaktion dar. Nach weiteren 4 Minuten wird die letzte Messung durchgeführt; deren Differenz zum Startsignal stellt das Signal der Hauptreaktion dar.
Folgende Signale werden mit dem Behring-Nephelometer II (Behringwerke AG, Marburg, Deutschland, Best.Nr. O VI A ) für eine Eichkurve erzielt (die Maßeinheit für die Streulichtsignale ist hier Bit):

| | Signal der Vorreaktion | | Signal der Hauptreaktion | |
|---|---|---|---|---|
| Unterer Eichkurvenpunkt (Verdünnung 1: 10240) | 5 | Bit | 768 | Bit |
| Oberer Eichkurvenpunkt (Verdünnung 1: 640) | 566 | Bit | 6255 | Bit |
| Probe 1 (Normalurin, 1:5) | 2 | Bit | 35 | Bit |
| Probe 2 (Pathol. Urin, 1:5) | 20 | Bit | 1805 | Bit |
| Probe 3 (Serum, 1:5) | 4456 | Bit | 1895 | Bit |

Proben 1 und 2 zeigen das typische Verhalten von Proben, die unterhalb des Meßbereiches bzw. im Meßbereich des Testansatzes liegen. Probe 3 zeigt eine deutliche Reaktion in der Vorreaktion, wobei ein Signal entsteht, das oberhalb desjenigen des obersten Eichkurvenpunktes liegt. Dadurch kann ein Flag erzeugt werden, das z.B. einen weiteren Ansatz zur Quantifizierung des Ergebnisses mit einer höheren Probenverdünnung bewirkt. Im klassischen Ansatz ohne Vorreaktion erzeugt die Probe 3 mit bezogen auf den Meßbereich extrem hoher Analytkonzentration ein Signal, welches mit dem der Probe 2 oder sogar der Probe 1 vergleichbar ist. Im klassischen Ansatz würde deshalb eine falsch zu niedrige Ergebniszuweisung erfolgen.

### Beispiel 2 :

### Bestimmung von IgM im Serum

Die Durchführung erfolgt analog zur Messung in Beispiel 1 mit 50 µL einer 1:20 vorverdünnten Serumprobe (in N-Diluenz) ab, wobei als Reagenz 40 µL N-Antiserum gegen Human-IgM (Best. Nr. OSAT, Behringwerke AG) zum Einsatz kommt.

| Bestimmung von IgM im Serum | | |
|---|---|---|
| | Signal der Vorreaktion | Signal der Hauptreaktion |
| Unterer Eichkurvenpunkt | | |
| (Verdünnung 1 : 80) | 5 Bit | 60 Bit |
| Oberer Eichkurvenpunkt | | |
| (Verdünnung 1 : 2.5) | 460 Bit | 4494 Bit |
| | | |
| Probe 1 (Normalserum, 1:20) | 11 Bit | 700 Bit |
| | | |
| Probe 2 (IgM-Myelom, 1:20) | 4414 Bit | 5128 Bit |
| dto., Nachmessung 1:100 | 864 Bit | 4368 Bit |
| dto., Nachmessung 1:400 | 101 Bit | 2053 Bit |
| | | |
| Probe 3 (IgM-Myelom, 1:20) | 5438 Bit | > 16384 Bit |
| dto., Nachmessung 1:400 | 180 Bit | 2792 Bit |
| | | |
| Probe 4 (IgM-Myelom, 1:20) | 514 Bit | 4402 Bit |
| dto. Nachmessung 1:100 | 43 Bit | 1421 Bit |

Es gilt hier der gleiche Sachverhalt wie im Beispiel 1. Durch sukzessive Verdünnung der Probe bis zu dem Zustand, in dem der Wert der Vorreaktion innerhalb bzw. unterhalb des korrespondierenden der Kalibrationskurve und der Meßwert der Probe in der Hauptreaktion in den in der Eichkurve vorgegebenen Werten zu liegen kommt, kann eine Quantifizierung von Problemproben mit extrem hoher Analytkonzentration erreicht werden. Während IgM-Myelom Proben 2 und 3 auch durch Überschreitung des Meßbereichs der Hauptreaktion richtig erkannt worden wären, ist dies beim IgM-Myelom 4 nicht mehr der Fall. Hier erfolgt eine Quantifizierung durch die automatische Nachbestimmung als Folge der Überschreitung des Schwellenwertes in der Vorreaktion.

## Patentansprüche

1. Immunchemisches Verfahren zur Bestimmung eines Analyten in einer Probe eines biologischen Materials durch eine analytvermittelte Präzipitationsreaktion mit analytspezifischen Bindungspartnern, das folgende Schritte einschließt:
a) Zugabe eines Aliquots der zu untersuchenden Probenmenge zu einem Reagenz, das mindestens einen analytspezifischen Bindungspartner enthält, der auch an ein Partikel gebunden sein kann und Durchführung einer ersten Inkubation;
b) erste Detektion der analytabhängigen Präzipitationsreaktion mittels eines geeigneten Analysengerätes;
c) Zugabe der restlichen Probenmenge zum Inkubationsansatz gemäß Schritt a) und Durchführung einer zweiten Inkubation;
d) zweite Detektion der analytabhängigen Präzipitationsreaktion mittels besagten Analysengerätes;
e) Bestimmung der Analytkonzentration:
i) durch Auswertung des während der ersten Detektion erhaltenen Meßsignals, sofern dieses oberhalb eines Schwellenwertes liegt; oder
ii) durch Auswertung des während der zweiten Detektion erhaltenen Meßsignals, sofern das während der ersten Detektion erhaltene Meßsignal unterhalb besagten Schwellenwertes liegt.

2. Vertahren gemäß Anspruch 1, **dadurch gekennzeichnet, daß** anstelle der Bestimmung der Analytkonzentration gemäß Schritt e) i) das Verfahren gemäß Anspruch 1 mit reduziertem Probenanteil wiederholt wird und die Bestimmung der Analytkonzentration durch Auswertung des während der zweiten Detektion der Wiederholung erhaltenen Meßsignals erfolgt.

3. Verfahren gemäß Anspruch 1, worin das Aliquot einem Anteil von 1 - 25 % der Gesamtprobe entspricht.

4. Verfahren gemäß Anspruch 3, worin das Aliquot einem Anteil von 2 - 20 % der Gesamtprobe entspricht.

5. Verfahren gemäß Anspruch 4, worin das Aliquot einem Anteil von 5 - 10 % der Gesamtprobe entspricht.

6. Verfahren gemäß Anspruch 1, wobei der analytspezifische Bindungspartner ein Antikörper ist.

7. Verfahren gemäß Anspruch 1, wobei die Messung der analytabhängigen Präzipitationsreaktion mittels turbidimetrischer Messung erfolgt.

8. Verfahren gemäß Anspruch 1, wobei die Messung der analytabhängigen Präzipitationsreaktion mittels nephelometrischer Messung erfolgt.

9. Verfahren gemäß Anspruch 1, wobei die Messung der analytabhängigen Präzipitationsreaktion mittels kinetischer Messung erfolgt.

10. Verfahren gemäß Anspruch 1, wobei die Messung der analytabhängigen Präzipitationsreaktion mittels einer fixed-time kinetischen Messung erfolgt.

11. Verfahren gemäß Anspruch 1, wobei die Messung der analytabhängigen Präzipitationsreaktion mittels einer fixed-value Messung erfolgt.

12. Verfahren gemäß Anspruch 1, wobei die Messung der analytabhängigen Präzipitationsreaktion mittels Endpunktmessung erfolgt.

13. Verfahren gemäß Anspruch 1, wobei die Probe unverdünnt eingesetzt wird.

14. Verfahren gemäß Anspruch 1, wobei die Probe vorverdünnt wird.

15. Verfahren gemäß Anspruch 1, wobei die Auswertung mittels Vergleich der gefundenen Meßwerte mit einer Eichkurve erfolgt.

16. Verfahren gemäß Anspruch 1, wobei die Auswertung mittels Vergleich mit einer Eichkurve erfolgt, die entweder unter den Bedingungen der ersten Reaktion (Schritte a und b) oder unter den Bedingungen der zweiten Reaktion (Schritte c und d) aufgenommen worden ist.

## Claims

1. An immunochemical method for determining an analyte in a sample of a biological material by an analyte-mediated precipitation reaction with analyte-specific binding partners, which includes the following steps:
a) addition of an aliquot of the amount of sample to be investigated to a reagent which comprises at least one analyte-specific binding partner, which can also be bound to a particle, and carrying out a first incubation;
b) first detection of the analyte-dependent precipitation reaction by means of suitable analysis equipment;
c) addition of the remaining amount of sample to the incubation mixture from step a) and carrying out a second incubation;
d) second detection of the analyte-dependent precipitation reaction by means of said analysis equipment;
e) determination of the analyte concentration:
i) by evaluating the measured signal obtained during the first detection, if it is above a threshold; or
ii) by evaluating the measured signal obtained during the second detection, if the measured signal obtained during the first detection is below said threshold.

2. The method as claimed in claim 1, wherein, in place of determination of the analyte concentration in step e) i), the method as claimed in claim 1 is repeated with reduced sample content, and the analyte concentration is determined by evaluating the measured signal obtained during the second detection of the repeat.

3. The method as claimed in claim 1, in which the aliquot corresponds to a proportion of 1-25% of the whole sample.

4. The method as claimed in claim 3, in which the aliquot corresponds to a proportion of 2-20% of the whole sample.

5. The method as claimed in claim 4, in which the aliquot corresponds to a proportion of 5-10% of the whole sample.

6. The method as claimed in claim 1, where the analyte-specific binding partner is an antibody.

7. The method as claimed in claim 1, where the measurement of the analyte-dependent precipitation reaction takes place by means of turbidimetric measurement.

8. The method as claimed in claim 1, where the measurement of the analyte-dependent precipitation reaction takes place by means of nephelometric measurement.

9. The method as claimed in claim 1, where the measurement of the analyte-dependent precipitation reaction takes place by means of kinetic measurement.

10. The method as claimed in claim 1, where the measurement of the analyte-dependent precipitation reaction takes place by means of a fixed-time kinetic measurement.

11. The method as claimed in claim 1, where the measurement of the analyte-dependent precipitation reaction takes place by means of a fixed-value measurement.

12. The method as claimed in claim 1, where the measurement of the analyte-dependent precipitation reaction takes place by means of endpoint measurement.

13. The method as claimed in claim 1, where the sample is used undiluted.

14. The method as claimed in claim 1, where the sample undergoes preliminary dilution.

15. The method as claimed in claim 1, where the evaluation takes place by comparing the measurements found with a calibration plot.

16. The method as claimed in claim 1, where the evaluation takes place by comparing with a calibration plot which has been recorded either under the conditions of the first reaction (steps a and b) or under the conditions of the second reaction (steps c and d).

## Revendications

1. Procédé immunochimique pour la détermination d'un analyte dans un échantillon d'un matériel biologique par une réaction de précipitation, médiée par analyte, avec des partenaires de liaison spécifiques d'analyte, qui comprend les étapes suivantes :
a) addition d'une partie aliquote de la quantité d'échantillon à analyser, à un réactif qui contient au moins un partenaire de liaison spécifique d'analyte, qui peut également être fixé à une particule, et exécution d'une première incubation;
b) première détection de la réaction de précipitation dépendant de l'analyte, au moyen d'un appareil d'analyse approprié ;
c) addition de la quantité restante d'échantillon au mélange d'incubation selon l'étape a) et exécution d'une seconde incubation ;
d) seconde détection de la réaction de précipitation dépendant de l'analyte, au moyen dudit appareil d'analyse ;
e) détermination de la concentration de l'analyte :
I) par évaluation du signal de mesure obtenu pendant la première détection, lorsque celui-ci se trouve au-dessus d'une valeur seuil ; ou
II) par évaluation du signal de mesure obtenu pendait la seconde détection, lorsque le signal de mesure obtenu pendant la première détection se trouve au-dessous de ladite valeur seuil.

2. Procédé selon la revendication 1, **caractérisé en ce qu'**au lieu de la détermination de la concentration de l'analyte selon l'étape e) i) on répète le procédé selon la revendication 1 avec une quantité réduite d'échantillon et on effectue la détermination de la concentration de l'analyte par évaluation du signal de mesure obtenu pendant la seconde détection de la répétition.

3. Procédé selon la revendication 1, dans lequel la partie aliquote correspond à une quantité de 1 à 25 % de l'échantillon total.

4. Procédé selon la revendication 3, dans lequel la partie aliquote correspond à une quantité de 2 à 20 % de l'échantillon total.

5. Procédé selon la revendication 4, dans lequel la partie aliquote correspond à une quantité de 5 à 10 % de l'échantillon total.

6. Procédé selon la revendication 1, dans lequel le partenaire de liaison spécifique d'analyte est un anticorps.

7. Procédé selon la revendication 1, dans lequel la mesure de la réaction de précipitation dépendant de l'analyte s'effectue au moyen d'une mesure turbidimétrique.

8. Procédé selon la revendication 1, dans lequel la mesure de la réaction de précipitation dépendant de l'analyte s'effectue au moyen d'une mesure néphélométrique.

9. Procédé selon la revendication 1, dans lequel la mesure de la réaction de précipitation dépendant de l'analyte s'effectue au moyen d'une mesure cinétique.

10. Procédé selon la revendication 1, dans lequel la mesure de la réaction de précipitation dépendant de l'analyte s'effectue au moyen d'une mesure cinétique à temps fixé.

11. Procédé selon la revendication 1, dans leqùel la mesure de la réaction de précipitation dépendant de l'analyte s'effectue au moyen d'une mesure à valeur fixée.

12. Procédé selon la revendication 1, dans lequel la mesure de la réaction de précipitation dépendant de l'analyte s'effectue au moyen d'une mesure à point final.

13. Procédé selon la revendication 1, dans lequel l'échantillon est utilisé non dilué.

14. Procédé selon la revendication 1, dans lequel l'échantillon est prédilué.

15. Procédé selon la revendication 1, dans lequel l'évaluation s'effectue au moyen d'une comparaison des valeurs trouvées avec une courbe d'étalonnage.

16. Procédé selon la revendication 1, dans lequel l'évaluation s'effectue au moyen d'une comparaison avec une courbe d'étalonnage qui a été construite dans les conditions de la première réaction (étapes a et b) ou dans les conditions de la seconde réaction (étapes c et d).
